(19) Europäisches Patentamt
European Patent Office
Office européen des brevets

(11) **EP 4 450 033 A1**

(12) **EUROPEAN PATENT APPLICATION**

(43) Date of publication:
**23.10.2024 Bulletin 2024/43**

(21) Application number: **23168840.9**

(22) Date of filing: **20.04.2023**

(51) International Patent Classification (IPC):
**A61F 13/15** (2006.01) **A61F 13/534** (2006.01)
**A61F 13/537** (2006.01)

(52) Cooperative Patent Classification (CPC):
**A61F 13/15658; A61F 13/15699; A61F 13/534;
A61F 13/537; A61F 13/53743**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Zampollo, Fabio
26013 Crema (IT)**

(72) Inventor: **Zampollo, Fabio
26013 Crema (IT)**

(74) Representative: **Plischke, Manfred
Ahornweg 18
61449 Steinbach Ts. (DE)**

(54) **ABSORBENT COMPOSITE WEB COMPRISING HIGH POROSITY LAYER AND PARTICLES INTERMINGLED WITH CONTINUOUS MICRO-FILAMENTS**

(57) The present invention relates to an absorbent composite web wherein particles intermingled with continuous micro-filaments are positioned within the pores of a high-loft web and to a process for forming such webs, which may be applied in filtration or - especially if the particles are liquid absorbing particles - in absorbent hygiene articles.

Fig. 3

## Description

## Field of the invention

The present invention relates to an absorbent composite web wherein particles intermingled with continuous micro-filaments are positioned within the pores of a high-loft web and to a process for forming such webs, which may be applied in filtration or - especially if the particles are liquid absorbing particles - in absorbent hygiene articles.

## Background

Webs comprising particulate materials are broadly used such as without limitation in air purification, where particles remove contaminants by adsorption mechanism. A further broad use relates to absorbent structures for absorbent articles, such as, without limitation absorbent articles for personal hygiene such as disposable baby diapers, training pants for toddlers or adult incontinence undergarments, are designed to absorb and contain body exudates, in particular urine. These absorbent articles comprise several layers providing different functions, typically including a topsheet, a backsheet and in between an absorbent core, among other layers. The absorbent core should be able to absorb and retain liquid exudates for a prolonged amount of time, for example overnight for a diaper, minimize re-wet to keep the wearer dry, and avoid soiling of clothes or bed sheets. Modern absorbent cores typically comprise absorbent structures comprising a composite of superabsorbent polymers (SAP) particles, also called absorbent gelling materials (AGM), and fibrous materials, as may be natural, such as cellulose fibers, modified natural, such as regenerated cellulose-based materials, or synthetic fibers.

It is well known to form suitable absorbent structures "in-line" or "in situ" on a converting line for forming the complete absorbent article, see e.g., WO2022/120693A1 (P&G) describing an absorbent core for use in absorbent article, comprising a liquid-permeable top cover layer, bottom cover layer and high loft central layer between top and bottom cover layers, and first and second superabsorbent polymers, which at least partially penetrate into high loft central layer, further comprising cover layers, which are adhesively attached. Also, WO2014/001487 (C4S) describes particles that are embedded in a porous web and (e.g.,) ultrasonically immobilized between cover webs.

However, such approaches require that each article production line, also referred to as converter, is equipped with appropriate handling systems for the particle addition, as well as unwinding and splicing systems for the preformed webs. Further, the formation of the absorbent core may be limiting the overall production volume.

Alternatively to the in-line core forming, composite absorbent webs comprising particles, such as superabsorbent particles, may be formed off-line at high production volumes, which can be delivered as so-called roll stocks to the article forming converter lines for being confectioned and/or combined with other elements to form the absorbent articles, thereby advantageously simplifying the converting equipment and process, as well as offering manufacturing cost advantages due to high production volumes.

WO2021/188330A1 (P&G) describes an absorbent core for use in an absorbent article, comprises liquid-permeable top layer, bottom layer, high-loft central layer, and superabsorbent polymer particles at least partially distributed within the central layer.

WO2020/025401 (BASF) describes a fluid-absorbent core comprising at least an upper layer and a lower layer, each layer comprising from 0 to 10 w-% fibrous material (natural or synthetic) and from 90 to 100 w-% water-absorbent polymer particles. The absorbent core is made by dropping a first water-absorbent polymer or a blend onto one side of a nonwoven material. A top tissue paper is then adhesively laminated, upon which the composite is turned around and the second water-absorbent polymer is dropped onto the other side of the nonwoven, also covered by adhesively laminating a lower tissue. Then, the absorbent composite is slit to the desired width and wound. However, there is still a need for improved approaches with regard to the structure, such as avoiding glues so as to ease processing at the recycling of factory and post-consumer scrap and to reduce the ecological footprint of such products. Also, the application of glues implies significant energy usage. Further, liquid handling may be negatively impacted by the use of adhesives in absorbent structures.

Also approaches avoiding the use of glue are known, such as from WO2013/152809 (TWE/Libeltex), wherein particles are embedded into the pores of a preformed web. For satisfactory particle containment, such loaded webs are enveloped in a separate step in wrap materials, such as tissues or non-woven webs.

WO2020/103964A1 (Reifenhäuser & PFN) describes an open porous spunbonded web of continuous crimped fibers, wherein at least a portion of the pores can be filled with particles, such as superabsorbent particles. Even when using crimped fibers, which leads to reduced manufacturing speed, the thickness thereof results in poor entrapment, requiring a further envelope web, as well as relatively low softness.

WO2021/198894 (GDM) describes a web comprising elastomeric melt-blown fibers, optionally also comprising non-elastomeric melt-blown filaments, with particulate material being trapped within the pores of the web and at least partly adherent to the fibers due to the stickiness of these upon melt blowing. Optionally, such a composite may be combined with auxiliary webs, such as non-wovens to retain the particles or high-loft materials for better liquid distribution during use. This approach is employing non-continuous melt-blown fibers as formed by "air-knife" technology, wherein the polymeric filaments are contacted at the outlet of the forming nozzle with attenuating air from an angled direction, thereby cutting the filaments into short pieces, which have a reduced particle containment functionality. Also, the need for using elastomeric polymers increases cost and reduces production flexibility and speed, as may not be acceptable.

Overall, whilst the off-line forming of the absorbent structures provides advantages over the in-line forming, such as very high production volume with one production unit, there is still a need for improving the economics and/or the properties of the resulting absorbent structures. Further, the use of adhesives in the absorbent structure complicates recycling, in particular factory scrap recycling and may negatively impact on consumer perception as an unnecessary chemical. Henceforth, the present invention aims at providing economic advantages by combining high production volume with one production unit and less costly raw materials and in particular without adhesives, without compromising on particle containment before and during manufacturing, but also during use.

## Summary

The present invention is a continuous absorbent composite web comprising filament-particle intermixture comprising intermingled polymeric filaments and particles, high-loft polymeric fiber matrix comprising pores, and scrim layer of essentially continuous polymeric filaments, which are essentially continuous. At least one, preferably two layers of the filament particle intermixture is/are positioned in contact with the high-loft filament matrix and at least a portion of the filament intermingled particles is penetrating into pores of the high-loft fiber matrix. At least one, preferably two scrim layers of essentially continuous filaments form the surface layer of the continuous absorbent composite web.

The continuous absorbent composite web preferably satisfies one or more of the conditions selected from the group consisting of

a) the filaments are coaxial meltblown filaments;
b) the composite web is essentially adhesive free;
c) the composite web is essentially cellulose free;
d) the scrim layer(s) adhere(s) to respective adjacent inwardly positioned layer in the absence of adhesive.
e) the particles are present at an amount of more than about 80% preferably ore than about 90% even more preferably of more than about 95%, based on the total weight of the composite web;
f) the particles are present in an amount of more than about 50 g/m$^2$, preferably more than about 100 g/m$^2$, more preferably of more than about 200 g/m$^2$, or most preferably of more than about 300 g/m2;
g) a single scrim layer (130) exhibits a basis weight of less than about 10 g/m$^2$, preferably less than about

5 g/m$^2$, more preferably less than about 3 g/m$^2$;
h) the high-loft polymeric fiber matrix exhibits a basis weight amount of more than about 20 g/m$^2$, preferably more than about 40 g/m$^2$, more preferably of more than about 50 g/m$^2$;
i) the polymeric filaments intermingled with the particles comprise more than about 95% of polypropylene exhibiting a MFR (at 230°C and 2.16kg) of more than about 25 g/ 10 mins, preferably more than about 45 g/ 10 mins.
j) the composite web comprises particle free longitudinal regions;
k) the high-loft polymeric matrix comprises sub-matrices, preferably sub-layers, that exhibit a difference of at least 10% relative to the higher value of at least one property selected form the group consisting of

k1) density;
k2) fiber or filament thickness;
k3) fiber or filament composition.

l) particle containment at a dry state of more than about 90%, preferably more than about 95%, more preferably of more than about 98%, and most preferably of more than about 99%.

In another aspect, the present invention is a process for forming a continuous absorbent composite web,

comprising the step of providing

◦ at least one, preferably two scrim forming co-axial meltblowing (CAM) unit(s);
◦ a first filament-particle intermixing unit, comprising

a first polymer supply and a first CAM filament forming unit,
and a first particle supply unit;
preferably a further polymer supply and a further CAM filament forming unit;

◦ optionally a second filament particle-intermixing unit (1200"), comprising

a second polymer supply and a second CAM filament forming unit,
a second particle supply unit,
preferably a further polymer supply and a further CAM filament forming unit,

◦ a pre-formed web unwinding unit as a high-loft web supply unit;
◦ at least one moving collection unit, preferably a moving foraminous belt system;
◦ optionally a web turning unit, whereby the gravitationally upper and lower surfaces of a web or web composite are interchanged;

further executing the following process steps

A - Forming a scrim of continuous filaments by a scrim forming CAM unit and feeding the first scrim towards the collection unit;
B - forming a filament-particle intermixture and feeding the intermixture towards the collection unit;
C - Providing a high-loft web

by unwinding a pre-formed web on a high-loft web unwinder or
by forming a high loft web, preferably by a spunbonding process from a polymer, preferably a polyester,
and feeding the high-loft web towards the collection unit,

X - optionally a turning step of inverting the gravitational orientation of a web or a composite web; in the order selected from the group consisting of

- A', C, B, A"
- A', B', C, B", A";
- C, B', A', X, B", A"; and

whereby A' and B' denote a first execution of the respective step, and A", B" a second execution of the respective step.

The process may be operated at a production volume as the product of production width [in m] times production speed [in m/min] of more than about 500 $m^2$/min, preferably more than 1000 $m^2$/min, more preferably of more than 2000 $m^2$/min, even more preferably of more than 4000 $m^2$/min, most preferably of more than 5000 $m^2$/min or [$m^2$/min].

The process for forming a continuous absorbent composite web may further comprise a step of working the particles of the filament-particle intermix into the high-loft material, preferably selected from the group consisting of

- air suctioning;
- mechanical vibrating;
- applying ultrasonics.

**Brief description of the Figures**

Fig. 1A and B depict an absorbent composite web according to the present invention in a partly exploded and non-exploded view.
Fig. 2A and B depict another absorbent composite web according to the present invention in a partly exploded and non-exploded view.
Fig. 3 shows a set up of an equipment suitable for a process according to the present invention.
Fig. 4A to C depict processing options according to the present invention.

The figures are schematically only, and not to scale. Same numerals refer to same or equivalent features or elements, single (') or multiple (", "', ...) apostrophes indicate duplicate features, such a left and right or front and back, etc. All percentages (%) refer to weight percentages unless expressly stated.

**Detailed description**

Within the present context, the term "fiber" refers to elongated fibers exhibiting a length to diameter ratio of at least about 3:1, often more than 10:1, or even over 100:1. Synthetic or man-made natural material-based fibers are typically formed from solidifying continuous filaments of molten polymers, as may be homogenous or monocomponent polymers, or polymers mixtures, or which may form distinct cross-sectional regions within a filament, whereby the latter may create crimped or crimpable fibers. The term "filament" is also interchangeably used for essentially continuous solidified fibers, whilst "fibers" are used to describe dis-continuous structures. A typical process for forming a web from essentially continuous filaments is known as "spunbonding", see e.g., US5935512 (K-C), creating essentially continuous filaments of a diameter in the range of about 1 to 50 $\mu$m, often between 15 to 35 $\mu$m.

"Micro-fibers" is used to describe fibers as made by meltblowing web formation as known from e.g., US8017534 (K-C), whereby low viscosity polymers are extruded through a nozzle and attenuated by a high velocity airstream blown at an angle relative to the formed filament. This scatters the melt, solidifies it and breaks it up into a fibrous web

More recently, co-axially meltblowing (CAM) technology has gained particular interest, providing essentially continuous micro filaments. Such CAM filament forming is described in more detail in e.g., US9303334 (Biax) and represents an important element for the present invention, as will be discussed in more detail herein below.

Whilst the polymers for such filaments can be made from a wide selection, preferred selections are polyolefins, and even more preferred ones polypropylene. Whilst elastomeric polyolefins may be employed, this is not necessary from a performance point of view and less preferred from a commercial point of view due to their increased cost. If any thereof be employed, this should be at a level of less than 5 w-%, based on the total weight of the filament polymer. Most preferred polymers exhibit a Melt Flow Index MFR from 25 MFR of more than about 25 g/10 mins, preferably more than about 45 g/ 10 mins, typically less than about 2000 g/10 mins, as may be determined by ASTM D1238 and ISO 1133, and for polypropylene as a polymer that suitably can be processed with the current equipment and process, it is suitably expressed in units of gram per 10 minutes at 210°C and 2.16 kg load.

Within the present context, a "web" comprises a matrix of single type of fibers or filaments, or of a mixture, as may be directionally or randomly orientated, and bonded by friction, and/or adhesion, and/or cohesion, whereby the latter may be imparted directly after filament formation, e.g., at the lay-down step of fiber or filament forming. Typically, a web is self-supporting and allows handling on manufacturing or converting equipment, though just a sub-web of a composite web may not have sufficient integrity alone. A web may comprise particles as a filament-particle-intermixture. A "composite web" refers to a combination of (sub-)webs in a layered configuration, and a "continuous web" is essentially endless in the length or x-direction corresponding during manufacturing to machine direction (MD), as may be wound on rolls, or spools, or "festooned" in boxes, which may be spliced together to form the essentially endless web. Thus, a continuous web exhibits a width perpendicular to the length direction, corresponding to the cross-direction during manufacturing, as may extend over several meter during manufacturing of the web, or over less than one meter and corresponding to the width as being used in the converting or forming of articles, and a thickness, perpendicular to the length and width, and significantly smaller than either of the two.

A further important element for the present invention is a high loft web. The term "high loft" refers to low density bulky fabrics, as compared to flat, paper-like fabrics. High loft webs are characterized by a relatively high porosity. This means that there is a relatively high amount of void space between the fibers in which particles, such as superabsorbent polymer particles, can be distributed. The high loft web (without the superabsorbent particles) suitable for the present invention may have a density at a pressure of 0.83 kPa (0.12 psi) below 0.15 $g/cm^3$, in particular ranging from 0.01 $g/cm^3$ to 0.15 $g/cm^3$, or from 0.05 $g/cm^3$ to 0.12 $g/cm^3$ or from 0.08 $g/cm^3$ to 0.10 $g/cm^3$. Preferably, the high-loft web also maintains its openness at increased pressure of 4.14kPa (0.6 psi) at a density of less than about 0.20 $g/cm^3$, in particular ranging from 0.01 $g/cm^3$ to 0.20 $g/cm^3$, or from 0.05 $g/cm^3$ to 0.15 $g/cm^3$, whereby the density can be calculated by dividing the basis weight of the high loft layer by its thickness measured at the respective pressure as indicated The basis weight and the thickness of a suitable high loft can be adjusted to the specifics of the particular application. The high loft (sub-)web may in particular have a thickness of at least 0.30 mm, in particular ranging from 0.30 mm to 2.00 mm, or from 0.50 mm to 1.5 mm, as measured at a pressure of 4.14 kPa (0.6 psi). The basis weight of the high-loft (sub-)web may for example range from 15 $g/m^2$ to 500 $g/m^2$, in particular from 30 $g/m^2$ to 200 $g/m^2$, for example 50 $g/m^2$ to 120 $g/m^2$.

The fibers forming the high-loft web may be made partially or entirely of a relatively resilient synthetic fibers, in particular polypropylene (PP), polyamide (PA, such as nylons) or polyethylene terephthalate (PET) fibers. The diameter of the fibers may for example range from 0.01 mm to 0.50 mm.

A particular exemplary execution of a high-loft web may be a through-air bonded carded web made from staple fibers that are sent through a combing or carding unit, which separates and generally aligns the staple fibers in the machine direction to form a generally machine direction-oriented fibrous nonwoven web. This web is then drawn through a heated drum, creating bonds throughout the fabric without applying specific pressure (through air bonding process).

Optionally, the high-loft web may comprise sub-web layers, exhibiting different properties and also different functionalities. Such properties may be density, fiber thickness, or fiber composition, and the difference in properties should be more than about 3%, or more than about 5 or even more than about 10%, based on the respective higher value.

A third important element for the present invention is particles that are to be positioned within the pores of the high-loft web.

Such particles may provide a wide range of functionalities, such as coloring, or scouring, or adsorption of gases or gas contaminants for filtration purposes. A particular application relates to the absorption of liquids, whereby the particles are adapted to absorb liquids, such as water or aqueous solutions, such as bodily exudates, at multiples of their own weight. "Superabsorbent polymers" (SAP) refers herein to absorbent materials that can absorb at least 10 times their weight of an aqueous 0.9% saline solution as measured using the Centrifuge Retention Capacity (CRC) test (EDANA method NWSP 241.0.R2 (19)). The SAP preferably have a CRC value of at least 15 g/g. SAP are typically water-insoluble but water-swellable cross-linked polymers capable of absorbing large quantities of fluids. SAP are in particulate form so as to be flowable in the dry state. Typical particulate SAP are polyacrylate polymers, however it is not excluded that other polymer materials may also be used. For example, starch-based particulate absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile.

SAP may be polyacrylates and polyacrylic acid polymers that are internally and/ or surface cross-linked. The superabsorbent polymer of the invention may be selected from polyacrylates and polyacrylic acid polymers that are internally and surface cross-linked. The superabsorbent polymers can be internally cross-linked, i.e., the polymerization is carried out in the presence of compounds having two or more polymerizable groups which can be free-radically copolymerized into the polymer network. Preferably, the SAP particles comprise crosslinked polymers of polyacrylic acids or their salts or polyacrylates or derivatives thereof.

The particles may be relatively small (under 1 mm in their longest dimension) in their dry state and may be roughly circular in shape, but granules, fibers, flakes, spheres, powders, platelets and other shapes and forms are also known to persons skilled in the art. Spherical-like particles may ease penetration into the pores of a high-loft web.

In a composite web according to the present invention the particles are intermingled with the polymeric micro-filaments, thus forming a filament-particle intermixture. This is easy to understand when considering how such an intermixture may be produced, namely by feeding a stream of particles into the attenuation zone of a filament forming apparatus, as will be discussed in more detail herein below. Thus, the filaments entangle the particles whilst collectively travelling towards a collecting belt. As the open porous high-loft web is already positioned onto the collecting belt, the intermixture is depositing onto the high-loft web and at least a portion of the filament-particle intermixture is penetrating into the pores of the high-loft web, optionally supported by a penetration aiding step, such as vibrating or air suctioning.

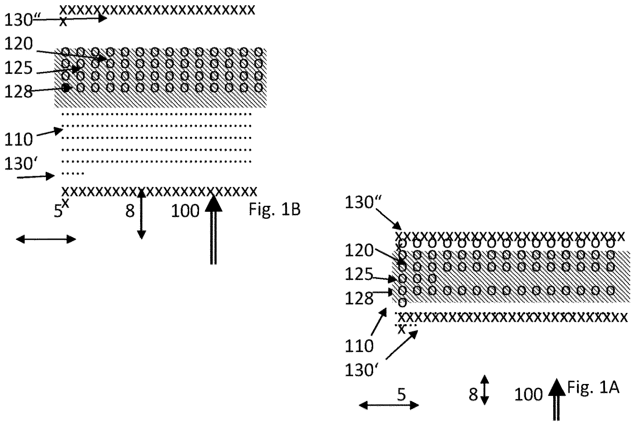

Having described the key elements of a continuous absorbent composite web reference is now made to Fig. 1A and B, showing such a web 100 in a cross-sectional view and in an exploded view for further explanations, as should not be seen limiting.

A high-loft web 110 is positioned next to a first, here lower, scrim layer 130'. Further shown is a filament-particle intermixture 120 of Particles 125 and filaments. Here it is important that the filaments are continuous CAM filaments 128, as discontinuous meltblown fibers and even more spunbonding filaments do not provide sufficient particle entanglement. In Fig. 1A, the filament-particle intermixture 120 is shown overlapping with the high-loft layer 110, indicating that at least a portion of the particles 125 penetrates into the pores of the high loft web 110. A second, here upper, scrim layer 130" is positioned on top of the combined intermixture / high-loft web. It should be noted that the composite web as described does not require any addition of adhesive or glue, as the in-situ-formed filaments provide sufficient tackiness to keep the structure together, and in particular to contain the Particles.

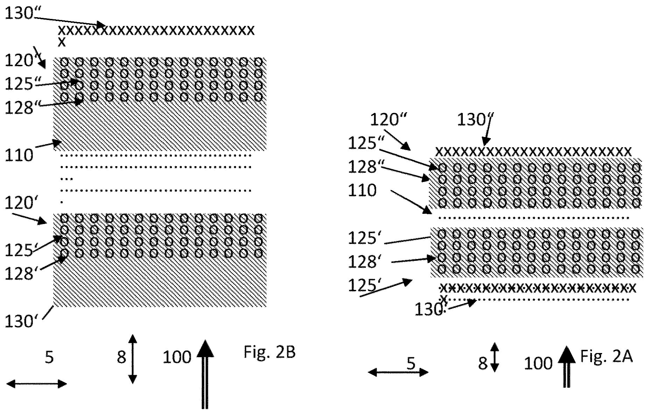

Fig. 2 A and B depict exemplarily a further execution for a composite web 100 according to the present invention, analogous to the one as shown in Fig. 1, except for a second filament-particle intermixture 120" in addition to first on 120', such that Particles penetrate from both sides into the high-loft layer 110.

For either of the executions, the Particles may, but do not need to, completely penetrate into the high-loft web 110, but a portion of the high-loft web 110 may be free of particles. This may be particularly preferred, if the x-y-directional fluid distribution should be enhanced.

The containment of the particles can be determined by a shake test, as described herein below. Preferably, at least about 90%, or more than about 95%, or more than about 98% or more than about 99% of the weight of the added particles are retained in the composite web after executing the test. It should be noted that such a good containment performance is achieved without the need for an additional containment web, such as a tissue or a preformed nonwoven, as enabled by the combined function of the filament-particle embedding into the pores of the high-loft web and the coverage of the scrim layer(s).

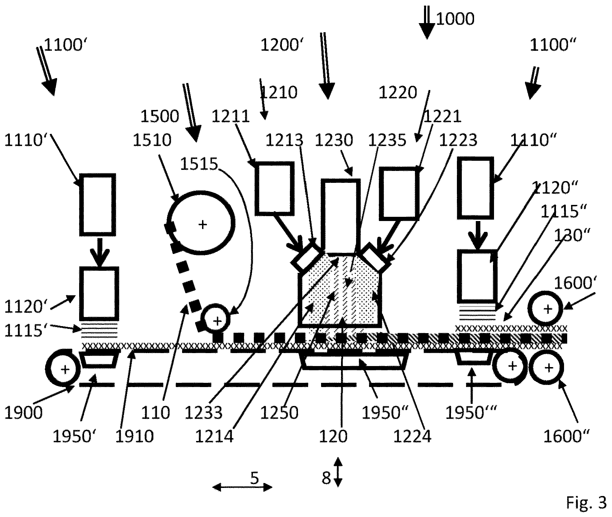

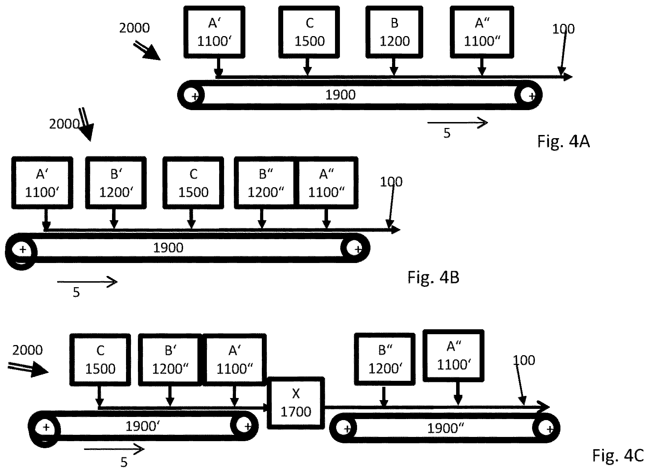

Referring now to Fig. 3 and 4, processing options for forming a continuous absorbent composite web 100 are described.

In a first exemplary execution as depicted in Fig.3, the process is executed on a continuous absorbent composite web forming unit 1000, which comprises a first (1100') and an optional second (1100") scrim forming CAM unit(s), such as known and described in more detail in the above referenced {...}. Each of the scrim forming units 1100 comprises a scrim polymer supply unit 1110 and a filament forming die head 1120 for forming the filaments 1115.

As shown for this execution, a high-loft web 110 is provided from an unwinder 1510 of a high-loft web supply 1500.

A first, and in this execution only, filament-particle intermixing unit 1200' comprises a first particle supply unit 1230', a first polymer supply 1211' for filaments being formed by a first CAM filament forming unit 1213', and a first particle supply unit 1230'. Optionally, though for many executions preferably, the filament particle intermixing unit comprises a further polymer supply 1221' and a further CAM filament forming unit 1223' to be positioned opposite to the first CAM filament forming unit 1213', relative to the entry of the particle infeed 1233. A collection unit 1900, preferably a foraminous belt system with respective suction devices 1950 is positioned gravitationally below the sub-web forming units.

As further depicted in Fig. 4A to C, the process 2000 according to these executions of the present invention is a combination of the process steps operated on the above equipment with the following abbreviation:

In process steps A, A', A", a scrim web is formed by the above-described scrim forming units 1100, 1100', or 1100", respectively.
In process steps B, B', B", filament-particle intermixes are formed on the described filament particle intermixing units 1200, 1200', 1200", respectively.
In process step C, a high-loft web 110 is provided from a high-loft supply unit 1500 by unwinding a preformed web 110 from a high-loft web unwinder 1510 and guiding it via guide roll 1515 towards the collecting unit, or by forming a high loft web 110 in-situ on the collection unit 1900, preferably by a spunbonding process from a polymer, preferably a polyester.

It should be noted, that any of the respective second steps A", B" may executed at differing process settings then the respective first one A', B'.

Thus, for this execution, the steps for providing a continuous absorbent composite web 100 as depicted in Fig.1 are executed relative to the machine direction as defined by the movement of the collection unit 1900 in the order A' - C - B - A", see Fig. 4A.

For providing a continuous absorbent composite web 100 as depicted in Fig. 2, the process steps may be executed relative to the machine direction as defined by the movement of the collection unit 1900 in the order A' - B' - C - B" - A", see Fig. 4B.

In a further variant, as depicted in Fig 5C, essentially the same structure as in Fig. 2 can be made with a further option of including a further process step X of inverting the gravitational orientation of a sub-web on a turning unit 1700.

Without wishing to be bound by the theory, it is believed that this variant enhances the penetration of the particles into the high loft web even more. To this end, the process starts with process step C of providing a high-loft web 110 on a high-loft supply unit 1500 and feeding the web on a first collection unit 1900' towards a first particle filament intermixing unit B' 1220' for positioning the filament-particle intermixture on said high-loft web. Aided by gravity and supporting suction by an appropriate suction unit 1950 of the collection unit 1900', the particles are deeply penetrating into the pores of the high-loft web 110. Thereafter, a first scrim is applied as step A' by a scrim forming unit 1100' on the surface of the combined high-loft and filament particle intermixture.

The next step X of turning the web "up-side down" can be made by conventional means on a web turning unit 1700, as may be a duo of web supports, such as parallel belts, being twisted by 180°. When the sub-web is further fed to a further web support unit 1900", the upper surface of the high-loft material faces upwardly and a second filament-particle intermixture can be deposited thereon as process step B" on a filament-particle intermixing unit 1220", further covers by a second scrim application step A" on a scrim forming unit 1100".

Thus, the process can be described by the process steps C - B' - A' - X - B" - A", see Fig. 4C.

It is a particular benefit of the process according to the present invention that it exploits the tackiness of the in-situ formed filaments to provide integrity of the structure and containment of the particles without the need for additional glue to be added.

Further, the process allows very high production volumes for the resulting continuous absorbent composite web, as it can be operated at very high production speed of more than about 500 m/min, or more than about 700 m/min, or even more than about 1000 m/min.

Further, the web width can be more than about 1 m, or more than 3 m, or more than 5 m, or more than 7 m, such that overall production rates of more than 500 $m^2$/ min, or more than 1000 $m^2$/min, or more than 2000 $m^2$/min, or more than 4000 $m^2$/min, or even more than 5000 $m^2$/min can be reached.

**Test methods.**

Shake-out test

The susceptibility of a particle containing fibrous web or matrix to the migration and escape of particles can be measured by employing a Shake-out Test procedure which involves agitating samples in a controlled fashion and determining the total loss of mass from the sample. A sample of the matrix or composite is prepared in the shape of a rectangular plate which has a length of 9 inch (22.86 cm) and a width of 4 inch (10.16 cm). The sample has the structure and as being incorporated in its intended end-product article.

The Shakeout Test can be conducted by employing a Model # RX-24 PORTABLE SIEVE SHAKER (herein after referred to as "RX-24") available from W.S. Tyler Inc., U.S.A. For use in the Shake-out Tests, the RX-24 is modified to shake samples and allow a determination of the web's resistance to the migration of particles, based on the material lost during the shaking. A sample holder has a frame made of polyacrylate plate and two pieces of mesh screen. The frame has a length of 17 inch (43.18 cm), a width of 11.5 inch (29.21 cm) and a thickness of 0.20 inch (0.51 cm). The frame has a rectangular opening with a length of 15.25 inch (38.74 cm) and a width of 6.25 inch (15.88 cm), and the opening is substantially centered in the frame. One piece of mesh screen with a dimension slightly larger than the opening is operatively joined on each side of the frame (e.g., with duct tape) to hold the test sample. The mesh screen had 0.4 cm x 0.4 cm square openings, and the total weight of the sample holder is about 500 grams. A substantially equivalent shaker system may optionally be employed.

To perform the Shake-out Test, the absorbent composite sample is laid at the center of the sample holder, and the sample holder is laid horizontally flat (i.e., parallel to the floor) upon the wire screen employed to support the sample on the modified RX-24. The RX-24 then shakes the sample at a frequency of approximately 520 cycles per minute for a period of five minutes. After the completion of the shaking portion of the test, the mass loss and the superabsorbent-loss are determined by comparing the total remaining mass of the absorbent composite sample with the original mass of the sample when the sample was initially placed on the support screen, in accordance with the following formula:

$$\text{Mass loss (\%)} = 100\% \times ((M_0 - M_{end}) \div M_0)$$

where: $M_0$ = sample mass prior to shakeout test (e.g. grams); $M_{end}$ = sample mass remaining after test (e.g. grams).

Mass that is lost from the sample will generally fall through the openings in the support screen. Any mass that remains on the screen is counted as mass loss. The shake-out value (%) is the total mass loss (%) produced at the above-described shaking conditions.

While the foregoing discussion has described in detail one desirable method for conducting the Shakeout test using a specific type of apparatus, it will be appreciated that those skilled in the art will be able to prepare other apparatus that will allow equivalent testing in which agitation applied to webs will yield the identical results in terms of loss as that achieved by the disclosed Shake-out Test.

Centrifuge Capacity Test

This test is generally following the Edana NWSP 241.0.R2 (15) Polyacrylate Superabsorbent Powders - Determination of the Fluid Retention Capacity in Saline Solution by Gravimetric Measurement Following Centrifugation, but applying it to an absorbent article, as per Test Method PA07/05 of Hygiene-Technologie GmbH (Hy-Tec), Haan, Germany. To this end, a test sample as treated according to the free swell absorption test is positioned within a commercial centrifuge exerting a centrifugal force of about 250 times gravity (e.g., by having an internal diameter of 225 mm at a rotation of 1400 rpm) for 3 mins. The test specimen is removed and weighed and the centrifuge absorbent capacity recorded in grams.

**Claims**

1.  A continuous absorbent composite web (100)

    comprising

    filament-particle intermixture (120) comprising intermingled polymeric filaments (128) and particles (125);
    high-loft polymeric fiber matrix (110) comprising pores;
    scrim layer of essentially continuous polymeric filaments (130),

    **characterized in that**

    said polymeric filaments (128, 130) are essentially continuous,
    at least one, preferably two layers of said filament particle intermixture (120) is/are positioned in contact with said high-loft filament matrix (110) and at least a portion of said filament intermingled particles (125) is penetrating into pores of said high-loft fiber matrix(110);
    at least one, preferably two scrim layers of essentially continuous filaments (130) form the surface layer of said continuous absorbent composite web (100).

2.  A continuous absorbent composite web according to claim 1, further satisfying one or more of the conditions selected from the group consisting of

    a) said filaments are coaxial meltblown filaments;
    b) said composite web is essentially adhesive free;
    c) said composite web is essentially cellulose free;
    d) said scrim layer(s) adhere(s) to respective adjacent inwardly positioned layer in the absence of adhesive.

3.  A continuous absorbent composite web according to claim 1 or 2, further satisfying one or more of the conditions selected from the group consisting of

    e) said particles are present at an amount of more than about 80% preferably ore than about 90% even more preferably of more than about 95%, based on the total weight of the composite web;
    f) said particles are present in an amount of more than about 50 $g/m^2$, preferably more than about 100 $g/m^2$, more preferably of more than about 200 $g/m^2$, or most preferably of more than about 300 $g/m^2$;
    g) a single scrim layer (130) exhibits a basis weight of less than about 10 $g/m^2$, preferably less than about 5 $g/m^2$, more preferably less than about 3 $g/m^2$;
    h) said high-loft polymeric fiber matrix exhibits a basis weight amount of more than about 20 $g/m^2$, preferably more than about 40 $g/m^2$, more preferably of more than about 50 $g/m^2$;
    i) said polymeric filaments intermingled with said particles comprise more than about 95% of polypropylene exhibiting a MFR (at 230°C and 2.16kg) of more than about 25 g/ 10 mins, preferably more than about 45 g/ 10 mins.
    j) said composite web comprises particle free longitudinal regions;
    k) said high-loft polymeric matrix comprises submatrices, preferably sub-layers, that exhibit a difference of at least 10% relative to the higher value of at least one property selected form the group consisting of

       k1) density;
       k2) fiber or filament thickness;
       k3) fiber or filament composition.

4.  A continuous absorbent composite web according to any of the preceding claims, further satisfying one or more of the conditions selected from the group

consisting of I) particle containment at a dry state of more than about 90%, preferably more than about 95%, more preferably of more than about 98%, and most preferably of more than about 99%.

5. A process for forming a continuous absorbent composite web,

comprising the step of providing

◦ at least one, preferably two scrim forming co-axial meltblowing (CAM) unit(s) (1100, 1100', 1100");
◦ a first filament-particle intermixing unit (1200'), comprising

a first polymer supply (1211') and a first CAM filament forming unit (1213'), and a first particle supply unit (1230'); preferably a further polymer supply (1221') and a further CAM filament forming unit (1223'),

◦ optionally a second filament particle-intermixing unit (1200"), comprising

a second polymer supply (1211") and a second CAM filament forming unit (1213"), a second particle supply unit (1230"), preferably a further polymer supply (1221") and a further CAM filament forming unit (1223"),

◦ a pre-formed web unwinding unit (1510) as a high-loft web supply unit (1500);
◦ at least one moving collection unit (1900), preferably a moving foraminous belt system;
◦ optionally a web turning unit (1700), whereby the gravitationally upper and lower surfaces of a web or web composite are interchanged;

further executing the following process steps

A - Forming a scrim of continuous filaments (130) by a scrim forming CAM unit (1100') and feeding said first scrim towards said collection unit (1900);
B - forming a filament-particle intermixture (120) and feeding said intermixture towards said collection unit (1900);
C - Providing a high-loft web (110)

by unwinding a pre-formed web (110) on a high-loft web unwinder (1510) or by forming a high loft web (110), preferably by a spunbonding process from a polymer, preferably a polyester, and feeding said high-loft web (110) towards said collection unit (1900),

X - optionally a turning step (1700) of inverting the gravitational orientation of a web or a composite web;
in the order selected from the group consisting of

- A', C, B, A"
- A', B', C, B", A";
- C, B', A', X, B", A"; and

whereby A' and B' denote a first execution of the respective step, and A", B" a second execution of the respective step.

6. A process for forming a continuous absorbent composite web according to claim 5, operated at a production volume as the product of production width [in m] times production speed [in m/min] of more than about 500 $m^2$/min, preferably more than 1000 $m^2$/min, more preferably of more than 2000 $m^2$/min, even more preferably of more than 4000 $m^2$/min, most preferably of more than 5000 $m^2$/min [$m^2$/min].

7. A process for forming a continuous absorbent composite web according to claims 5 or 6, further comprising a step of working said particles of said filament-particle intermix into said high-loft material, preferably selected from the group consisting of

- air suctioning;
- mechanical vibrating;
- applying ultrasonics;
- ??.

8. A process for forming a continuous absorbent composite web according to any of claims 5 to 7, wherein respective second steps A", B" are executed at differing process settings then the respective first ones A', B'.

Fig. 1A

Fig. 1B

EP 4 450 033 A1

Fig. 2A

Fig. 2B

11

Fig. 3

Fig. 4A

Fig. 4B

Fig. 4C

## EUROPEAN SEARCH REPORT

Application Number

EP 23 16 8840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2021/198894 A1 (GDM SPA [IT]) 7 October 2021 (2021-10-07) * page 3, line 19 – line 25 * * page 4, line 23 – page 5, line 2 * * page 8, line 10 – page 9, line 6; claims 1–15; figure 1 * | 1-8 | INV. A61F13/15 A61F13/534 A61F13/537 |
| A,D | WO 2022/120693 A1 (PROCTER & GAMBLE [US]; YE FENGCHUN [CN]) 16 June 2022 (2022-06-16) * claims 1–15; figures 2, 8 * | 1-8 | |
| A,D | WO 2020/025401 A1 (BASF SE [DE]) 6 February 2020 (2020-02-06) * page 32, line 21 – page 36, line 31; figure 2 * | 1-8 | |

**TECHNICAL FIELDS SEARCHED (IPC)**

A61F
B32B

The present search report has been drawn up for all claims

| Place of search | Date of completion of the search | Examiner |
|---|---|---|
| Munich | 21 September 2023 | Demay, Stéphane |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 16 8840

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2023

| Patent document cited in search report | | Publication date | Patent family member(s) | | Publication date |
|---|---|---|---|---|---|
| WO 2021198894 | A1 | 07-10-2021 | CN | 115426993 A | 02-12-2022 |
| | | | EP | 4125755 A1 | 08-02-2023 |
| | | | JP | 2023519748 A | 12-05-2023 |
| | | | US | 2023142520 A1 | 11-05-2023 |
| | | | WO | 2021198894 A1 | 07-10-2021 |
| WO 2022120693 | A1 | 16-06-2022 | CN | 116583250 A | 11-08-2023 |
| | | | EP | 4259055 A1 | 18-10-2023 |
| | | | WO | 2022120693 A1 | 16-06-2022 |
| WO 2020025401 | A1 | 06-02-2020 | CN | 112512476 A | 16-03-2021 |
| | | | EP | 3829511 A1 | 09-06-2021 |
| | | | JP | 2021532865 A | 02-12-2021 |
| | | | US | 2021290453 A1 | 23-09-2021 |
| | | | WO | 2020025401 A1 | 06-02-2020 |

EPO FORM P0459

For more details about this annex : see Official Journal of the European Patent Office, No. 12/82

**EP 4 450 033 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022120693 A1
- WO 2014001487 A
- WO 2021188330 A1
- WO 2020025401 A
- WO 2013152809 A
- WO 2020103964 A1
- WO 2021198894 A
- US 5935512 A
- US 8017534 B
- US 9303334 B